# DEMANDE DE BREVET EUROPEEN

(11) **EP 1 380 274 A1**
(43) Date de publication de la demande: **14.01.2004**
(21) Numéro de dépôt: 03356104.4
(22) Date de dépôt: 04.07.2003
(51) Int. Cl.: A61F 2/40, A61F 2/32

(54) **Prothèse d'épaule ou de hanche facilitant l'abduction**

(30) Priorité: 05.07.2002 FR 0208500
(71) Demandeur: Société Anonyme TORNIER, F-38330 Saint Ismier (FR)
(72) Inventeur: Tornier, Alain, 38330 Saint-Ismier (FR)
(74) Mandataire: Schouller, Jean-Philippe

(57) **Abrégé**

Cette prothèse (P) comprend un composant huméral ou fémoral (1), présentant une surface (S₁, S₂) d'articulation concave, et un composant intermédiaire (3) présentant une première (S'₁) et une seconde (S'₂) surfaces d'articulation convexes destinées à coopérer respectivement avec ladite surface d'articulation concave (S₁) du composant (1) huméral ou fémoral et avec une surface d'articulation glénoïdienne ou cotyloïdienne concave (S₂). Le lieu (A₁) des centres instantanés (C₁) de rotation de la première surface d'articulation convexe (S'₁), par rapport à la surface d'articulation concave (S1) humérale ou fémorale, et le lieu (A₂) des centres instantanés de rotation de la seconde surface d'articulation convexe (S'₂) sur la surface glénoïdienne ou cotyloïdienne sont situés de part et d'autre de la première surface (S'₁).

## Description

L'invention a trait à une prothèse totale ou partielle d'épaule ou de hanche permettant de reproduire, avec un degré de précision amélioré, les caractéristiques d'une articulation naturelle.

Dans le domaine des prothèses d'épaule, il est connu, par exemple de la demande de brevet européen 0 299 889, de créer une surface articulaire convexe sur un composant glénoïdien, alors qu'une surface articulaire concave de forme correspondante est formée sur un composant huméral. Le composant glénoïdien d'une telle surface est très invasif et il peut se produire un conflit sous-acrominal du composant huméral en fin de mouvement d'abduction.

Il est par ailleurs connu du brevet américain 4,846,840 de réaliser, sur un élément intermédiaire d'une prothèse, deux surfaces convexes globalement concentriques en vue de leur articulation sur des surfaces concaves de formes correspondantes, prévues respectivement sur deux os à articuler l'un sur l'autre. Une telle prothèse est instable, notamment à cause du déport entre les deux jeux de surfaces articulaires prévus dans cette prothèse.

C'est à ces inconvénients qu'entend plus particulièrement remédier l'invention en proposant une prothèse articulaire d'épaule ou de hanche reproduisant l'articulation anatomique, tout en facilitant l'abduction du bras ou de la jambe, en l'absence de la coiffe des rotateurs pour l'épaule ou des structures stabilisatrices de la hanche, grâce à une augmentation du bras de levier de l'effort exercé par le deltoïde ou le moyen fessier en début d'abduction.

Dans cet esprit, l'invention concerne une prothèse d'épaule ou de hanche qui comprend un composant huméral ou fémoral présentant une surface d'articulation concave et un composant intermédiaire présentant une première et une seconde surfaces d'articulation convexes, destinées à coopérer respectivement avec la surface d'articulation concave du composant huméral ou fémoral et une surface d'articulation glénoïdienne ou cotyloïdienne concave, naturelle ou appartenant à un composant glénoïdien ou cotyloïdien. Cette prothèse est caractérisée en ce que le lieu des centres instantanés de rotation de la première surface d'articulation convexe par rapport à la surface d'articulation concave humérale ou fémorale et le lieu des centres instantanés de rotation de la seconde surface d'articulation convexe sur la surface d'articulation glénoïdienne ou cotyloïdienne sont situés de part et d'autre de la première surface convexe.

Grâce à l'invention, dans la cas d'une prothèse d'épaule, le bras de levier du deltoïde exerçant l'effort d'abduction de l'humérus sur l'épaule est important, ce qui facilite l'abduction grâce à un glissement de la surface d'articulation concave humérale par rapport à la première surface d'articulation convexe de l'élément intermédiaire. Dans le cas d'une prothèse de hanche, l'abduction du fémur, qui est commandée par le moyen fessier, est facilitée.

Selon des aspects avantageux de l'invention, cette prothèse incorpore une ou plusieurs des caractéristiques des revendications 2 à 12.

L'invention sera mieux comprise et d'autres avantages de celle-ci apparaîtront plus clairement à la lumière de la description qui va suivre de quatre modes de réalisation d'une prothèse conforme à son principe, donnée uniquement à titre d'exemple et faite en référence aux dessins annexés dans lesquels :
- la figure 1 est une coupe sagittale de principe d'une prothèse d'épaule conforme à l'invention en place sur un patient, alors que le bras du patient est en position basse ;
- la figure 2 est une coupe analogue à la figure 1 lors d'une première phase du mouvement d'abduction de l'humérus
- la figure 3 est une coupe analogue à la figure 1 lors d'une seconde phase du mouvement d'abduction ;
- la figure 4 est une coupe analogue à la figure 1 au terme du mouvement d'abduction ;
- la figure 5 est une coupe analogue à la figure 1, lorsque la prothèse est soumise à un effort tendant à l'écarter de sa position d'équilibre ;
- la figure 6 est une vue analogue à la figure 1, à plus petite échelle et avec le fémur représenté en vue extérieure, pour une prothèse de hanche conforme à un second mode de réalisation de l'invention ;
- la figure 7 est une coupe longitudinale de principe d'une prothèse d'épaule conforme à un troisième mode de réalisation de l'invention ;
- la figure 8 est une coupe selon la ligne VIII-VIII à la figure 7, on y a indiqué en VII-VII le plan de coupe de la figure 7 ;
- la figure 9 est une vue en perspective d'une partie du composant huméral de la prothèse des figures 7 et 8 ;
- la figure 10 est une vue analogue à la figure 7 pour une prothèse de hanche conforme à un quatrième mode de réalisation de l'invention ;
- la figure 11 est une coupe selon la ligne XI-XI à la figure 10, on y a indiqué en X-X le plan de coupe de la figure 10 et
- la figure 12 est une vue en perspective d'une partie du composant fémoral de la prothèse de la figure 10.

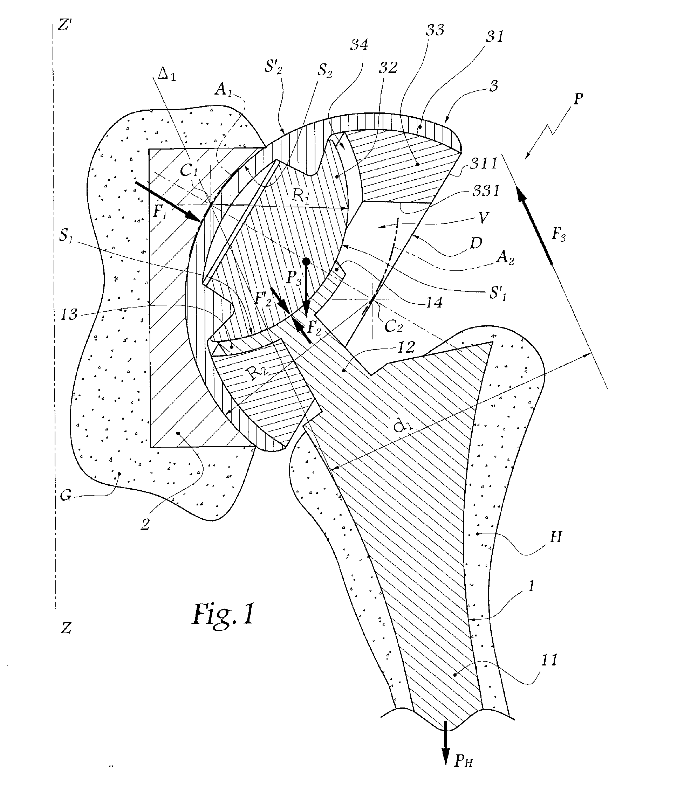

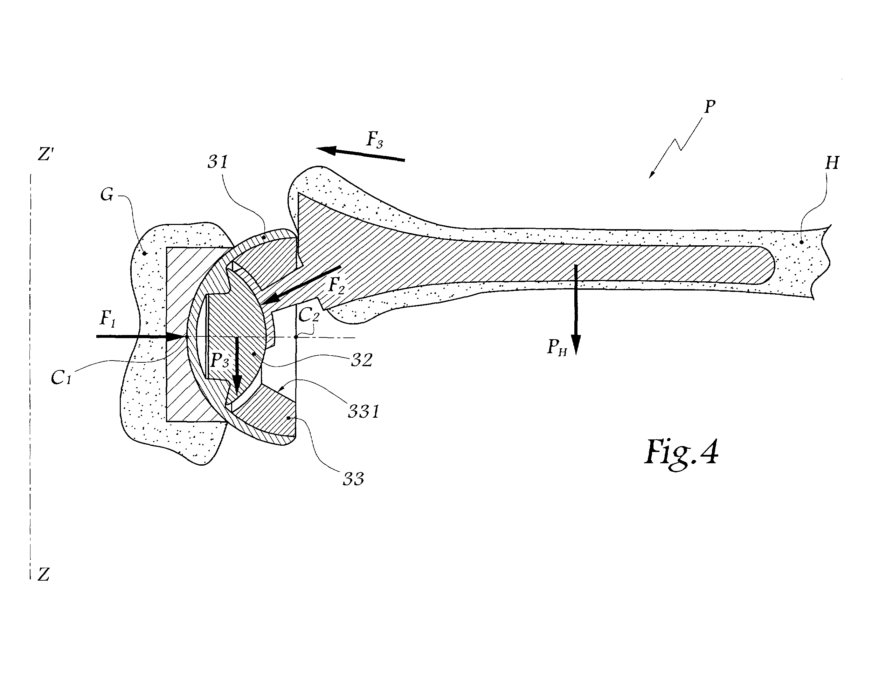

La prothèse P représentée aux figures 1 à 5 comprend un composant huméral 1 qui inclut une partie 11 destinée à être ancrée dans le canal médullaire de l'humérus H de l'articulation à équiper de la prothèse P. La partie 11 se prolonge par une tige 12 faisant saillie à l'extérieur de l'humérus une fois celui-ci réséqué et à l'extrémité de laquelle est ménagé un patin 13 monobloc avec les parties 11 et 12. Ce patin pourrait également être rapporté sur les parties 11 et 12.

Le patin 13 définit une surface concave S₁ dont la concavité est tournée vers la glène G de l'épaule.

La prothèse comprend également un composant glénoïdien 2 ancré dans la glène de l'épaule et définissant une surface concave S₂ dont la concavité est tournée vers l'extérieur de la glène.

Entre les composants 1 et 2 est intercalé un composant intermédiaire 3 composant une coupelle creuse 31 à l'intérieur de laquelle sont immobilisés un bouton 32 et une rondelle 33.

Les éléments 32 et 33 sont fixés par tout moyen approprié à l'intérieur de la coupelle 31, par exemple par vissage, emboîtage et/ou collage. En variante, le bouton 32 peut être monobloc avec la coupelle 31.

On note S'₁ la surface convexe du bouton 32 accessible depuis l'extérieur de la coupelle 31.

Les surfaces S₁ et S'₁ sont toutes deux des tronçons de sphère ayant sensiblement le même rayon R₁, de telle sorte que le patin 13 peut glisser sur la surface S'₁ du bouton 32.

La surface extérieure convexe S'₂ de la coupelle 31 est également en forme de tronçons de sphère, avec un rayon R₂ analogue au rayon de la surface S₂, ce qui permet un mouvement de glissement relatif des surfaces S₂ et S'₂.

Les éléments 32 et 33 sont logés dans un volume intérieur V de la coupelle 31, ce volume étant défini à l'intérieur de la surface S'₂ et d'un disque imaginaire D en appui sur le bord périphérique 311 de la coupelle 31. Selon une variante non représentée de l'invention, la rondelle 33 peut dépasser hors du volume V.

Le patin 13 est en forme de coupelle et s'étend autour de la tige 12 en formant une extension ou saillie périphérique circulaire 14 qui peut être engagée dans une encoche périphérique 34 ménagée, dans le volume V, entre le bouton 32 et la rondelle 33.

La rondelle 33 comprend une surface tronconique interne 331 contre laquelle peut venir en appui la tige 12, de telle sorte que la surface 331 constitue une butée au mouvement de coulissement du patin 13 par rapport au bouton 32. La surface 331 n'est pas nécessairement tronconique.

On note Z-Z' un axe vertical globalement parallèle à la colonne vertébrale du patient en station debout.

Le composant 3 est soumis au poids P₃, ainsi qu'à un effort F₁ qui lui est transmis par la glène. Cet ensemble est également soumis à un effort de réaction F₂ provenant du patin 13. Les effort P₃, F₁ et F₂ s'équilibrent en position de repos de l'humérus H.

Le composant 1 est soumis à son poids cumulé à celui de l'humérus, poids dont on note P_{H} la résultante. Le composant 1 est également soumis à la réaction du bouton 32, c'est-à-dire à un effort F'₂ opposé à l'effort F₂. Le composant 1 est enfin soumis à un effort F₃ exercé par le deltoïde soutenant l'humérus H.

Le centre C₁ de rotation instantané de la surface S₁ par rapport à la surface S'₁ est un centre commun aux sphères définissant les surfaces S₁ et S'₁. Compte tenu de la géométrie du composant 3, ce centre C₁ est situé dans la glène G, c'est-à-dire dans une position médiale par rapport au centre de rotation anatomique de l'épaule avant opération.

En pratique, le rayon de courbure R₁ des surfaces S₁ et S'₁ est choisi le plus grand possible, de telle sorte que le centre C₁ est le plus possible médialisé. La position du bouton 32 dans la coupelle 31 est également choisie dans ce but.

Par ailleurs, le centre instantané de rotation C₂ entre les surfaces S'₂ et S₂ est le centre commun des sphères contenant ces surfaces et il est situé au-delà de la surface S'₁ par rapport à la surface S₂. En pratique, le centre C₂ est sensiblement voisin du centre anatomique de rotation de l'épaule avant opération. La relation spatiale entre les centres C₁ et C₂ est donc une image de la relation spatiale entre le centre C₁ et le centre anatomique de rotation par rapport auquel sont implantés les muscles et les ligaments de l'épaule.

Ce positionnement des centres C₁ et C₂ permet de faciliter le mouvement d'abduction de l'humérus H, sans nécessiter que l'effort F₃ exercé par le deltoïde à cette occasion ne soit trop important.

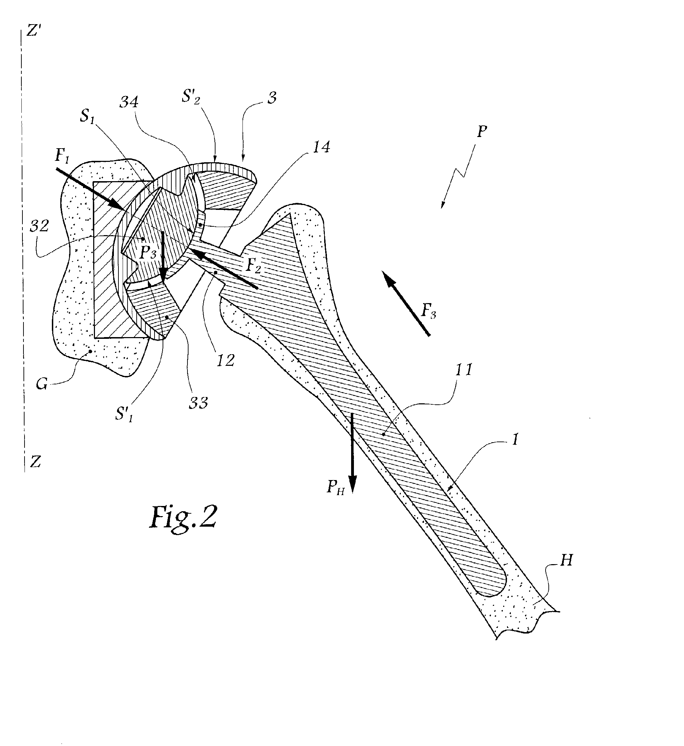

En effet, lors d'une première étape d'abduction représentée par le passage de la configuration de la figure 1 à celle de la figure 2, l'effort F₃ exercé par le deltoïde est exercé à une première distance d₁ relativement importante d'une droite Δ₁ parallèle à l'effort F₃ et passant par le centre C₁. Cette distance d₁ représente le bras de levier de l'effort F₃ par rapport au centre instantané de rotation C₁, ce bras de levier relativement important permettant de générer relativement facilement un mouvement de glissement du patin 13 sur le bouton 32, alors que la coupelle 31 demeure immobile par rapport au composant 2. Pendant cette première phase d'abduction, les forces de réaction F₁ et F₂ entre la glène et le composant 3 gardent approximativement la même direction, d'où un maintien en équilibre de la coupelle 31.

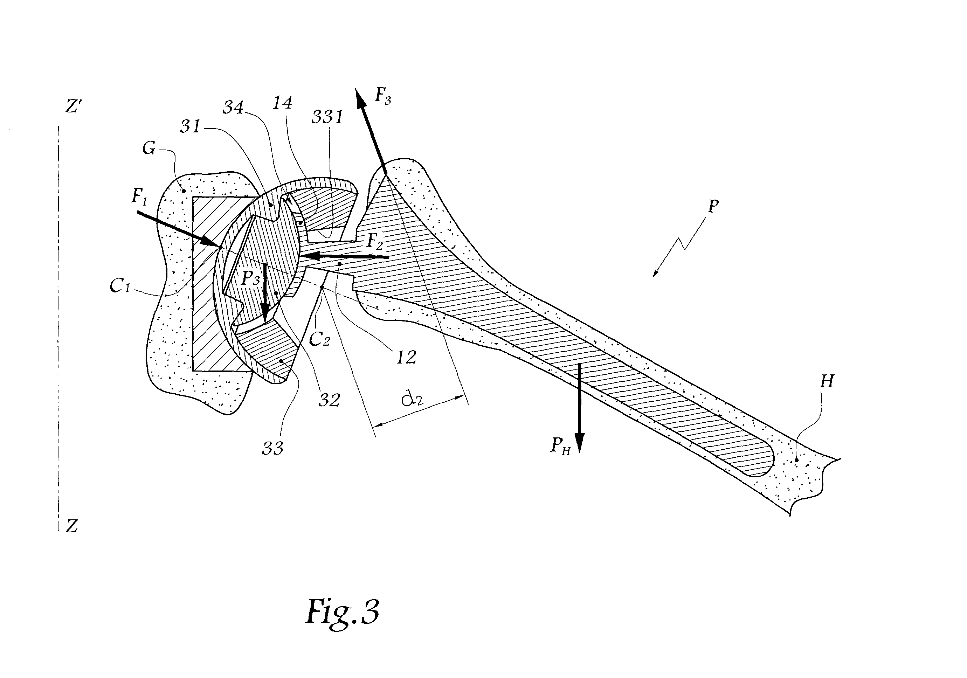

Si le mouvement d'abduction est prolongé jusqu'à atteindre la configuration de la figure 3, le patin 13 continue à glisser sur la surface S'₁ du composant intermédiaire 3, alors que la coupelle 31 entame un mouvement de glissement contre la surface S₂ du composant 2. En effet, pendant ce mouvement supplémentaire correspondant au passage de la configuration de la figure 2 à celle de la figure 3, les efforts F₁ et F₂ changent de direction.

Si l'on continue le mouvement d'abduction jusqu'à une ouverture maximale correspondant sensiblement à une position l'horizontale de l'humérus, la tige 12 peut venir en appui contre la surface 331 de la rondelle 33 au niveau de sa partie supérieure et le mouvement d'articulation a alors lieu uniquement par un déplacement de la coupelle 31 par rapport au composant 2. En pratique, la rondelle 33 a essentiellement pour fonction d'éviter un contact métal/métal entre le patin 11 ou la tige 12 et la coupelle 31.

Ainsi, le centre instantané de rotation de l'articulation n'est plus situé dans la glène, comme C₁, mais à l'intérieur de l'humérus, dans une position plus proche de celle du centre anatomique de rotation. Dans cette posture, la valeur du bras de levier d₂ entre l'effort F₃ exercé par le deltoïde et le centre C₂ est du même ordre de grandeur que valeur de d₁.

En pratique, le centre C₁ décrit, au cours du mouvement d'abduction, un arc de cercle A₁ pratiquement confondu sur les figures avec la trace des surfaces S₂ et S'₂ car les rayons R₁ et R₂ sont tels que le centre C₁ est pratiquement situé au niveau de ces surfaces. Cet arc cercle A₁ est le lieu de ces centres instantanés de rotation au cours du mouvement d'abduction. Le centre C₂ décrit un arc de cercle A₂ qui constitue le lieu des centres de rotation entre les surfaces S'₂ et S₂.

Le lieu A₁ des centres de rotation C₁ n'est pas nécessairement confondu avec la trace des surfaces S₂ et S'₂, cette configuration découlant simplement de la version représentée sur les figures. En pratique, le lieu A₁ est, le plus possible, médialisé et, par exemple, situé dans le composant 2 ou dans la glène G, ceci afin d'augmenter le bras de levier du deltoïde.

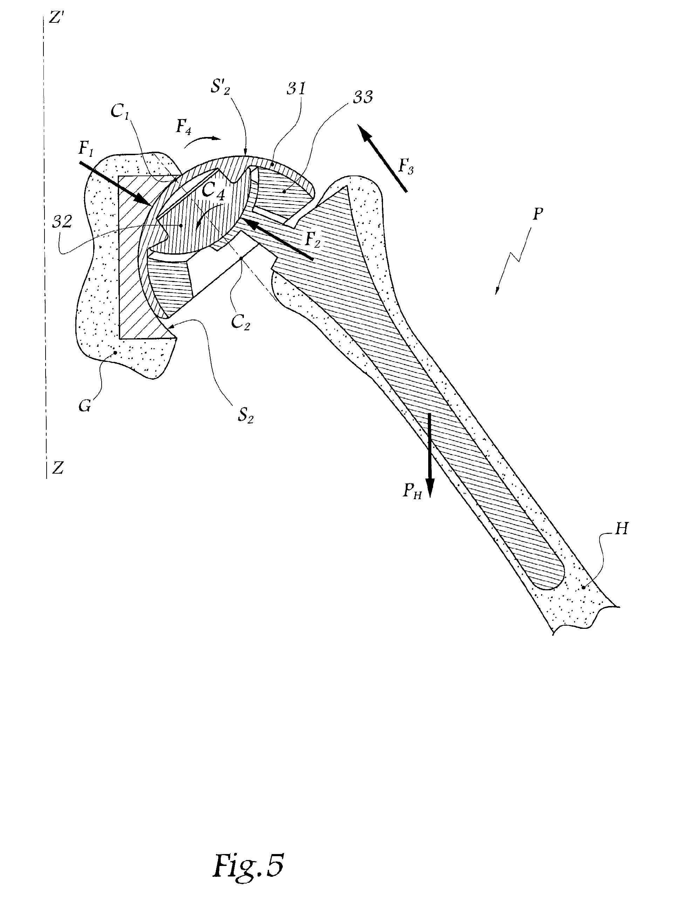

Comme il ressort plus particulièrement de la figure 5, la prothèse conforme à l'invention présente une grande stabilité dynamique. En effet si, à partir de la position de la figure 2 et dans des conditions de charge identiques, on déplace la coupelle 31 dans le sens de la flèche F₄, il se crée du fait du desaxage des efforts F₁ et F₂ un couple de rappel C₄ qui tend à ramener le composant 3 dans un sens opposé au déplacement F₄, créant ainsi les conditions d'un équilibre stable du composant 3.

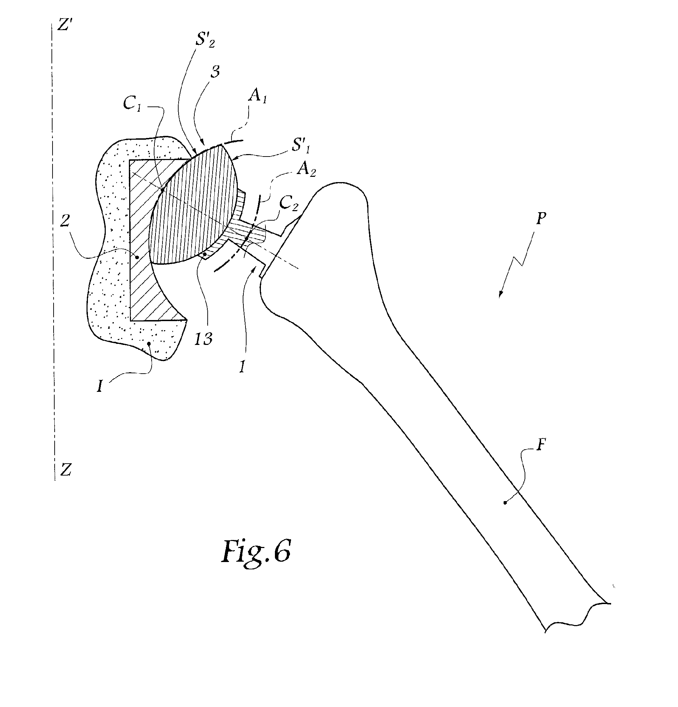

Dans le second mode de réalisation de l'invention représenté à la figure 6, les composants 1 et 2 sont respectivement destinés à être ancrés dans le fémur F et l'os iliaque I. Ils sont analogues à ceux décrits en référence au premier mode de réalisation. Le composant intermédiaire 3 diffère du précédent en ce qu'il a une forme globalement bi-convexe avec une première surface S'₁ en forme de tronçons de sphère dont on note C₁ le centre géométrique et une seconde surface S'₂, également en forme de tronçons de sphère, dont on note C₂ le centre géométrique. Les centres C₁ et C₂ sont les centres instantanés de rotation lors des mouvements de glissement du patin 13 du composant 1 par rapport au composant 3 et du composant 3 par rapport au composant 2.

En pratique, des forces de frottement non représentées doivent être vaincues lors des mouvements de l'humérus H ou du fémur F. Ces forces ont des valeurs faibles par rapport aux efforts mentionnés ci-dessus, ce qui permet de négliger les forces de frottement dans les explications qui précèdent.

En plus de la facilité du mouvement d'abduction qu'elle permet, la prothèse conforme à l'invention présente l'avantage particulier que les interventions à réaliser sur la glène ou sur l'os iliaque pour l'implantation du composant 2 sont limitées, voire nulles. En effet, la prothèse conforme à l'invention ne comprend pas nécessairement un composant glénoïdien ou cotyloïdien puisque la surface articulaire glénoïdienne ou cotyloïdienne peut être conservée si elle est en bon état. Dans le cas d'une prothèse totale telle que représentée sur les figures ci-jointes, le composant 2 a un volume faible, à la différence des composants correspondants de la majorité des prothèses de l'art antérieur.

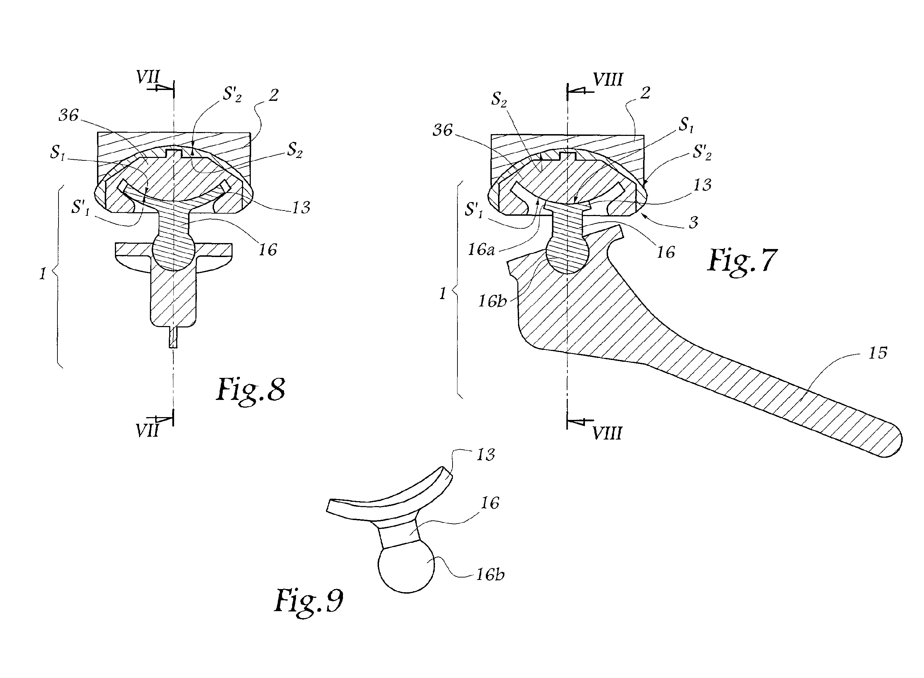

Dans le troisième mode de réalisation de l'invention représenté aux figures 7 à 9, les composants 1 et 2 sont respectivement destinés à être ancrés dans l'humérus et dans la glène. Le composant intermédiaire 3 définit deux surfaces articulaires convexes S'₁ et S'₂ destinées à coopérer respectivement avec des surfaces articulaires concaves S₁ et S₂ formées par les composants 1 et 2.

Les lieux des centres instantanés de rotation des surfaces S'₁ et S'₂ sur les surfaces S₁ et S₂ sont, comme précédemment, situés de part et d'autre de la surface S'₁.

Ce mode de réalisation diffère des précédents en ce que le composant huméral 1 n'est pas monobloc mais bipartite. Plus précisément, il comprend une queue humérale 15 et un doigt 16 dont une extrémité 16a forme le patin 13, alors que son autre extrémité est de forme globalement sphérique et reçue dans une cavité de forme correspondante de la queue 15.

Le patin 13 est globalement de forme ovale, avec sa plus petite dimension dans le plan de la figure 7, ce qui permet d'améliorer l'amplitude du mouvement d'abduction.

Comme il ressort de la figure 8, le mouvement transversal au plan sagittal est d'amplitude limitée.

La largeur du patin 13 dans le plan de la figure 8 évite une séparation intempestive des éléments 1 et 3.

On note qu'une seule pièce monobloc 36 joue le rôle du bouton 32 et de la rondelle 33 du premier mode de réalisation.

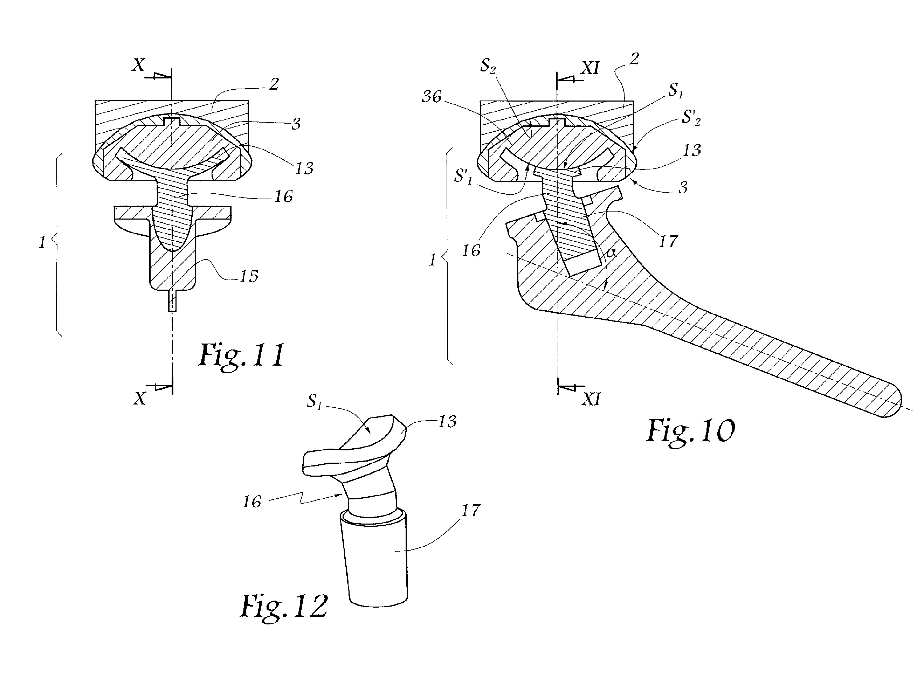

Dans le quatrième mode de réalisation de l'invention représenté aux figures 10 et 11, les composants 1 et 2 sont respectivement destinés à être ancrés dans le fémur et dans l'os iliaque. Le composant intermédiaire 3 est analogue à celui du troisième mode de réalisation et les surfaces convexes S'₁ et S'₂ qu'il définit induisent le même positionnement de leurs centres instantanés de rotation que précédemment.

Ce mode de réalisation diffère du précédent en ce que l'assemblage entre la queue fémorale 15 et le doigt 16 est conique. Une partie tronconique 17 est prévue sur le doigt 16 pour être engagée dans un logement de la queue 15.

En outre, le patin 13 qui définit la surface concave S₁ destiné à coopérer avec la surface S'₁ du composant 3 est de forme globalement rectangulaire. Comme dans le troisième mode de réalisation, la plus petite dimension du patin 13 est disposée dans le plan de la figure 10.

On note que le doigt 16 n'est pas rectiligne, ce qui permet d'optimiser la position du centre de rotation du patin 13. En pratique, l'angle α entre l'axe transversal à la surface S₁ et l'axe longitudinal de la queue 15 est choisi égal à environ 130°, ce qui présente un bon compromis entre la nécessité d'obtenir un maximum d'amplitude du mouvement d'abduction et la volonté de réduire le risque de luxation.

Selon un aspect non représenté de l'invention, le col reliant les parties 13 et 17 du doigt 16 peut être également coudé, dans le plan sagittal, dans une direction opposée à celle représentée. Ce col peut également être coudé dans un plan perpendiculaire au plan sagittal, ce qui permet de faire varier l'antéversion.

L'assemblage entre les éléments 15 et 16 peut être réversible. En d'autres termes, le doigt 16 peut éventuellement être démonté de la tige 15. Ce caractère réversible de l'assemblage des éléments 15 et 16 peut également être envisagé pour le troisième mode de réalisation.

L'invention a été représentée avec des surfaces articulaires en forme de tronçons de sphères. Elle est cependant applicable à d'autres types de surfaces, par exemple cylindriques à base circulaire ou paraboloïde, auquel cas la position des centres de rotation instantanée peut varier au cours du mouvement d'abduction sur des lieux qui ne sont pas nécessairement des arcs de cercle. De la même manière, on peut concevoir que les surfaces S₁ et S'₁ soient cylindriques, à génératrice rectiligne et à base circulaire, avec un axe globalement antéro-postérieur n'autorisant que le mouvement d'abduction, alors que les surfaces S₂ et S'₂ resteraient sphériques, en autorisant à la fois le mouvement d'abduction et la rotation axiale-humérale ou axiale-fémorale.

Sur les figures ci-jointes, les longueurs des flèches sont indicatives et ne doivent pas être considérées comme strictement représentatives des intensités des efforts correspondants. De même en est-il en ce qui concerne leur orientation.

L'invention a été représentée lors de sa mise en oeuvre avec des prothèses totales d'épaule et de hanche. Elle est cependant applicable avec une prothèse dépourvue de composant glénoïdien, la surface articulaire concave de la glène étant utilisée au lieu de la surface S₂ représentée sur les figures. De même, en est-il de même dans le cas d'une prothèse de hanche où la cavité cotyloïde naturelle peut être utilisée. Les caractéristiques des différents modes de réalisation représentés peuvent être combinées entre elles dans le cadre de la présente invention. En particulier, les prothèses des second et quatrième mode de réalisation pourraient être adaptées à l'épaule, alors que celles des premier et troisième mode de réalisation pourraient être adaptées à la hanche.

## Revendications

1. Prothèse d'épaule ou de hanche comprenant un composant huméral ou fémoral présentant une surface d'articulation concave et un composant intermédiaire présentant une première et une seconde surfaces d'articulation convexes destinées à coopérer respectivement avec ladite surface d'articulation concave dudit composant huméral ou fémoral et avec une surface d'articulation glénoïdienne ou cotyloïdienne concave naturelle ou appartenant à un composant glénoïdien ou cotyloïdien, **caractérisée en ce que** le lieu (A₁) des centres instantanés de rotation (C₁) de ladite première surface d'articulation convexe (S'₁), par rapport à la surface d'articulation concave humérale ou fémorale (S₁), et le lieu (A₂) des centres instantanés de rotation (C₂) de ladite seconde surface d'articulation convexe (S'₂) par rapport à ladite surface d'articulation glénoïdienne ou cotyloïdienne (S₂) sont situés de part et d'autre de ladite première surface d'articulation convexe (S'₁).

2. Prothèse selon la revendication 1, **caractérisée en ce que** ladite première surface d'articulation convexe (S'₁) est située à l'intérieur d'un volume (V) défini par ladite seconde surface d'articulation convexe (S'₂).

3. Prothèse selon l'une des revendications précédentes, **caractérisée en ce que** lesdites surfaces d'articulation (S₁, S₂, S'₁, S'₂) sont globalement en forme de tronçons de sphère.

4. Prothèse selon l'une des revendications 1 ou 2, **caractérisée en ce que** la première surface d'articulation convexe (S'₁) et la surface d'articulation concave humérale (S₁) sont cylindriques, à génératrice rectiligne et à base circulaire, avec leur axe de symétrie globalement antéro-postérieur par rapport à l'articulation, alors que la seconde surface d'articulation convexe (S'₂) et la surface d'articulation glénoïdienne (S₂) sont globalement en forme de tronçon de sphère.

5. Prothèse selon l'une des revendications précédentes, **caractérisée** en ce ledit composant intermédiaire (3) comprend une coupelle (31) formant ladite seconde surface d'articulation convexe (S'₂), un bouton (32) étant monobloc avec ladite coupelle ou immobilisé à l'intérieur de celle-ci et formant ladite première surface d'articulation convexe (S'₁).

6. Prothèse selon l'une des revendications précédentes, **caractérisée en ce que** ledit composant huméral ou fémoral (1) comprend un patin (13), formant la surface d'articulation concave (S₁) destinée à coopérer avec ladite première surface d'articulation convexe (S'₁), et une partie (11) destinée à être ancrée dans le canal médullaire huméral ou fémoral, ledit patin étant relié à ladite partie par une tige de liaison (12).

7. Prothèse selon la revendication 6, **caractérisée en ce que** ledit patin (13) est de forme non circulaire.

8. Prothèse selon la revendication 7, **caractérisée en ce que** la plus petite dimension dudit patin (13) est disposée parallèlement au plan sagittal (figures 7 et 10).

9. Prothèse selon l'une des revendications 1 à 4, 7 ou 8, **caractérisée en ce que** ledit composant intermédiaire (3) est de forme globalement bi-convexe.

10. Prothèse selon l'une des revendications précédentes, **caractérisée en ce qu'**elle comprend un composant (2) glénoïdien ou cotyloïdien formant ladite surface articulaire concave (S₂) glénoïdienne ou cotyloïdienne.

11. Prothèse selon l'une des revendications précédentes, **caractérisée en ce que** ledit composant huméral ou fémoral (1) est pourvu, au niveau de sa partie (13) formant ladite surface d'articulation concave (S₁), d'au moins une saillie (14) apte à être engagée dans un logement (34) de forme correspondante ménagée sur ledit composant intermédiaire.

12. Prothèse selon l'une des revendications précédentes, **caractérisée en ce que** ledit composant intermédiaire (3) comporte une rondelle (33) immobilisée dans une coupelle (31) formant ladite seconde surface d'articulation (S'₂), la surface interne (331) de ladite rondelle étant apte à limiter l'amplitude du déplacement relatif entre ledit composant huméral ou fémoral (1) et ledit composant intermédiaire.

13. Prothèse selon l'une des revendications précédentes, **caractérisée en ce que** ledit composant huméral ou fémoral (1) est bipartite et comprend une queue d'ancrage (15) sur laquelle est monté un élément (16) définissant ladite surface d'articulation humérale ou fémorale (S₁).
